# EUROPÄISCHE PATENTSCHRIFT

(11) **EP 1 289 365 B1**
(45) Veröffentlichungstag und Bekanntmachung des Hinweises auf die Patenterteilung: **08.09.2004**
(21) Anmeldenummer: 01964978.9
(22) Anmeldetag: 13.06.2001
(51) Int. Cl.: A01N 37/18, C07C 233/69, A01N 37/38, A01N 37/36, C07C 233/22

(54) **VERWENDUNG VON PHENETHYLACRYLAMIDEN, NEUE PHENETHYLACRYLAMIDE SOWIE HERSTELLUNGSVERFAHREN**
USE OF PHENETHYL ACRYLAMIDES, NOVEL PHENETHYL ACRYLAMIDES, METHOD FOR THE PRODUCTION THEREOF AND AGENTS CONTAINING THE SAME
UTILISATION DE PHENETHYLACRYLAMIDES, NOUVEAUX PHENETHYLACRYLAMIDES, LEUR PROCEDE DE PRODUCTION ET LES PRODUITS LES CONTENANT

(30) Priorität: 14.06.2000 DE 10028576
(43) Veröffentlichungstag der Anmeldung: 12.03.2003
(73) Patentinhaber: BASF AKTIENGESELLSCHAFT, 67056 Ludwigshafen (DE)
(72) Erfinder: GRAMMENOS, Wassilios, 67071 Ludwigshafen (DE); SAUTER, Hubert, 68167 Mannheim (DE); CULLMANN, Oliver, 64646 Heppenheim (DE); GEWEHR, Markus, 56288 Kastellaun (DE); MÜLLER, Bernd, 67227 Frankenthal (DE); TORMO I BLASCO, Jordi, 69514 Laudenbach (DE); GÖTZ, Norbert, 67547 Worms (DE); VOLK, Thorsten, 64297 Darmstadt (DE); LORENZ, Gisela, 67434 Hambach (DE); AMMERMANN, Eberhard, 64646 Heppenheim (DE); STIERL, Reinhard, 67112 Mutterstadt (DE); STRATHMANN, Siegfried, 67117 Limburgerhof (DE)
(86) Internationale Anmeldenummer: PCT/EP2001/006686
(87) Internationale Veröffentlichungsnummer: WO 2001/095721

(56) Entgegenhaltungen:
- WO-A-96/17825
- US-A- 3 657 340
- HIRAI Y ET AL: "A PHOTOCHEMISTRY OF 1-(1-PHENYLVINYL)-3,4-DIHUDROISOQUINOLINE" HETEROCYCLES, XX, XX, Bd. 25, 1987, Seiten 201-204, XP000566000 ISSN: 0385-5414

## Beschreibung

Die vorliegende Erfindung betrifft die Verwendung von Phenethylacrylamiden der Formel I 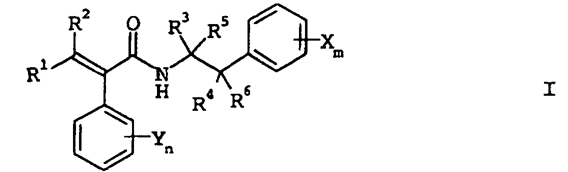 in der die Substituenten folgende Bedeutungen haben:
- X: Halogen, C₁-C₄-Alkyl, C₁-C₄-Halogenalkyl, C₁-C₈-Alkoxy, C₁-C₄-Halogenalkoxy und -O-C(R^{a},R^{b})-C≡C-R^{C};
R^{a},R^{b} unabhängig voneinander Wasserstoff und C₁-C₆-Alkyl;
R^{c} Wasserstoff, C₁-C₈-Alkyl, C₃-C₈-Cycloalkyl und Phenyl, welches substituiert sein kann durch Halogen, Cyano, Nitro, CF₃, C₁-C₄-Alkyl und/oder C₁-C₄-Alkoxy;
- m: 1 bis 4, wobei die Reste X verschieden sein können, wenn m größer als 1 ist;
- n: 1 oder 2, wobei die Reste Y verschieden sein können, wenn n=2 ist;
- Y: Halogen, Nitro, Cyano, C₁-C₄-Alkyl, CF₃, C₁-C₄-Alkoxy und Phenyl; wobei Y in 3-, 4- oder 3,4-Position steht;
- R¹,R²: unabhängig voneinander Wasserstoff, Halogen, C₁-C₄-Alkyl und CF₃;
- R³,R⁴,R⁵,R⁶: unabhängig voneinander Wasserstoff, C₁-C₄-Alkyl und C₁-C₄-Alkoxy oder
R³ und R⁴ gemeinsam einen Cyclopropylring bilden, wobei die C-R⁵- und die C-R⁶-Bindungen zueinander E- oder Z-ständig sein können;
zur Bekämpfung von pflanzenpathogenen Schadpilzen.

Außerdem betrifft die Erfindung neue Phenethylacrylamide, Verfahren zu deren Herstellung, sowie sie enthaltende Mittel. Verschiedene Arylacrylamide sind aus EP-A 407 217, EP-A 529 736, WO-A 93/01523, JP-A 06/080 616, US 3657340, US 3526653 und JP-A 63/154 649 bekannt. Sie werden z. T. als Herbizide beschrieben. Ihre fungizide Wirkung ist im Stand der Technik jedoch unbekannt.

Fungizide Acrylamide wurden in der nachveröffentlichten EP-A 1 295 868 beschrieben.

α-Oximinophenylessigsäurearylamide werden in WO-A 96/17825 und WO-A 96/23763 als Fungizide und in JP 02/200 658 als Herbizide beschrieben. Arylacrylamide werden in WO-A 96/17825 nur von der allgemeinen Offenbarung umfaßt.

Die fungizide Wirkung der in den vorstehend genannten Dokumenten beschriebenen ist jedoch in vielen Fällen nicht zufriedenstellend. Daher lag der Erfindung als Aufgabe zugrunde, Verbindungen mit verbesserter Wirkung zu finden.

Demgemäß wurde die Verwendung der Phenethylacrylamide der Formel I als Fungizide und die neuen Phenethylacrylamide, sowie sie enthaltende Mittel gefunden.

Die Phenethylacrylamide der Formel I sind auf den in EP-A 407 217, EP-A 529 736, WO-A 93/01523, JP-A 06/080 616, US 3657340, US 3526653 und JP-A 63/154 649 beschriebenen Synthesewegen herstellbar, deren Offenbarung hiermit einbezogen ist.

Die neuen Phenethylacrylamide, in denen R¹ und R² gleich sind und Cl, F und CH₃ bedeuten, sind beispielsweise ausgehend von α-Ketoestern der Formel II, in der R C₁-C₄-Alkyl bedeutet, auf den im Folgenden beschreibenen Wegen zugänglich:

Verbindungen, in denen R¹ und R² Chlor bedeuten, werden erhalten, in dem man α-Ketoester der Formel II mit Triphenylphosphin (PPh₃) und CCl₄ zu Acrylestern der Formel IIIa umgesetzt. Diese Umsetzung erfolgt üblicherweise bei Temperaturen von 10°C bis 120°C, vorzugsweise 20°C bis 80°C, in einem inerten organischen Lösungsmittel [vgl. Tetrahedron Lett., S. 3003ff., 1988].

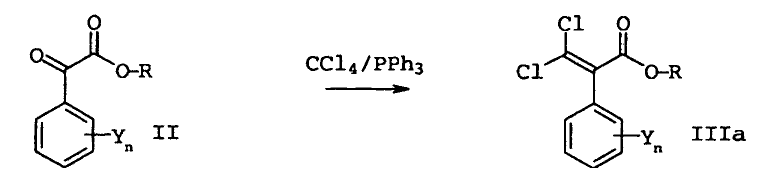

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Nitrile wie Acetonitril und Propionitril, sowie Dimethylsulfoxid, Dimethylformamid und Dimethylacetamid, besonders bevorzugt Acetonitril und Propionitril. Es können auch Gemische der genannten Lösungsmittel verwendet werden.

Die Edukte werden im allgemeinen in äquimolaren Mengen miteinander umgesetzt. Es kann für die Ausbeute vorteilhaft sein, CCl₄ und PPh₃ in einem Überschuß bezogen auf II einzusetzen.

Verbindungen, in denen R¹ und R² Fluor bedeuten, werden erhalten, in dem man α-Ketoester der Formel II mit Diphenyl-1,1-difluormethylphoshin der Formel VI, in der Ph für Phenyl steht, zu Acrylestern der Formel IIIb umgesetzt. Diese Umsetzung erfolgt üblicherweise bei Temperaturen von -70°C bis +80°C, vorzugsweise 0°C bis 20°C, in einem inerten organischen Lösungsmittel in Gegenwart einer Base [vgl. Tetrahedron Lett., S. 5571ff., 1990].

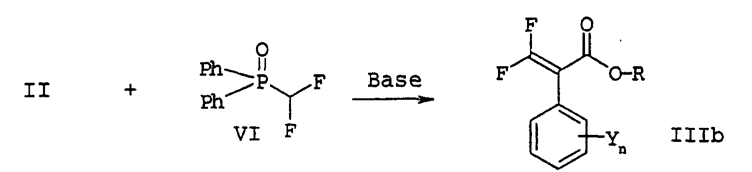

Geeignete Lösungsmittel sind Ether wie Diethylether, Diisopropylether, tert.-Butylmethylether, Dioxan, Anisol und Tetrahydrofuran, besonders bevorzugt Diethylether und Tetrahydrofuran Es können auch Gemische der genannten Lösungsmittel verwendet werden.

Als Basen kommen allgemein anorganische Verbindungen wie Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, metallorganische Verbindungen, insbesondere Alkalimetallalkyle wie Methyllithium, Butyllithium, Lithiumdiisopropylamin (LDA) und Phenyllithium in Betracht. Besonders bevorzugt werden Butyllithium und LDA.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, VI in einem Überschuß bezogen auf II einzusetzen.

Alternativ können Verbindungen, in denen R¹ und R² Fluor bedeuten, auch erhalten werden, in dem man α-Ketoester der Formel II mit Natrium-2-Chlor-2,2-difluoracetat der Formel VII und Triphenylphosphin (PPh₃) zu Acrylestern der Formel IIIb umgesetzt. Diese Umsetzung erfolgt üblicherweise bei Temperaturen von 20°C bis 180°C, vorzugsweise 60°C bis 180°C, in einem inerten organischen Lösungsmittel [vgl. Org. Synth. Bd. V, S. 949ff. (1973)].

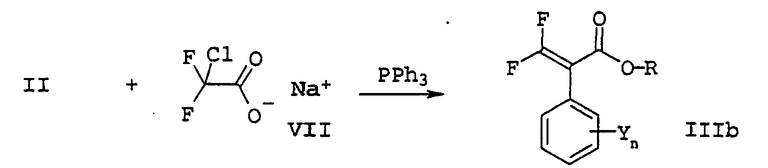

Geeignete Lösungsmittel sind aromatische Kohlenwasserstoffe wie Toluol, o-, m- und p-Xylol, Ether wie Diethylether, Diisopropylether, tert.-Butylmethylether, Dioxan, Anisol, Tetrahydrofuran (THF), Ethylenglycoldimethylether, Diethylenglycoldimethylether und 1,2-Diethoxyethan, Nitrile wie Acetonitril und Propionitril, sowie Dimethylsulfoxid, Dimethylformamid und Dimethylacetamid, besonders bevorzugt THF und Diethylenglycoldimethylether. Es können auch Gemische der genannten Lösungsmittel verwendet werden.

Die Edukte werden im allgemeinen in äquimolaren Mengen miteinander umgesetzt. Es kann für die Ausbeute vorteilhaft sein, VII in einem Überschuß bezogen auf II einzusetzen.

Verbindungen, in denen R¹ und R² Methyl bedeuten, werden erhalten, in dem man α-Ketoester der Formel II mit iso-Propylphosphoniumhalogenid der Formel VIII im Sinne einer Wittig-Reaktion umsetzt. Bevorzugt als Halogenide der Formel VIII sind Jodide und Bromide.

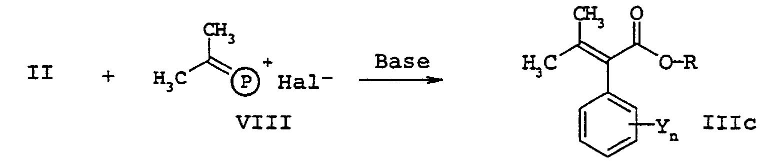

In dem voranstehenden Reaktionsschema steht 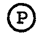 in der Formel VIII für einen Phosphoranylrest, wie beispielsweise Triphenylphosphoranyl.

Die Wittig-Reaktion erfolgt üblicherweise bei Temperaturen von -78°C bis +85°C, vorzugsweise -10°C bis +65°C, in einem inerten organischen Lösungsmittel in Gegenwart einer Base [vgl. Can. J. Chem. 1971, S. 2143ff.].

Geeignete Lösungsmittel sind aromatische Kohlenwasserstoffe wie Toluol, o-, m- und p-Xylol, Ether wie Diethylether, Diisopropylether, tert.-Butylmethylether, Dioxan, Anisol und Tetrahydrofuran (THF), Nitrile wie Acetonitril und Propionitril, sowie Dimethylsulfoxid, Dimethylformamid und Dimethylacetamid, besonders bevorzugt Diethylether und THF. Es können auch Gemische der genannten Lösungsmittel verwendet werden.

Als Basen kommen allgemein anorganische Verbindungen wie Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, metallorganische Verbindungen, insbesondere Alkalimetallalkyle wie Methyllithium, Butyllithium und Phenyllithium, Alkalimetall- und Erdalkalimetallalkoholate wie Natriummethanolat, Natriumethanolat, Kaliumethanolat, Kalium- tert.-Butanolat und Dimethoxymagnesium, in Betracht. Besonders bevorzugt werden Natriumhydrid und Natriummethanolat.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein,VIII in einem Überschuß bezogen auf II einzusetzen.

Carbonsäureester der Formel III, in denen R¹ und R² gleich sind und Cl, F und CH₃ bedeuten, werden nach üblichen Methoden zu den Carbonsäuren der Formel IV verseift [vgl. Organikum, 16. Aufl., S. 415 und 622, VEB Deutscher Verlag der Wissenschaften, Berlin 1985]. Diese Reaktion erfolgt üblicherweise bei Temperaturen von 10°C bis 80°C, vorzugsweise 20°C bis 60°C, in einem inerten organischen Lösungsmittel in Gegenwart einer Base, wie Alkali- oder Erdalkalihydroxiden, insbesondere Natrium- oder Kaliumhydroxid.

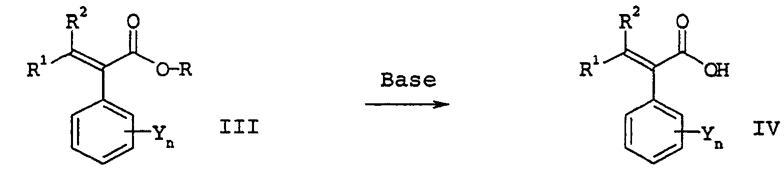

Carbonsäuren der Formel IV können in bekannter Weise direkt mit Phenethylaminen der Formel V zu den Verbindungen der Formel I amidiert werden [vgl. Houben-Weyl, Methoden der Organischen Chemie, Bd. E5, S. 941-972, Georg Thieme Verlag Stuttgart und New York 1985].

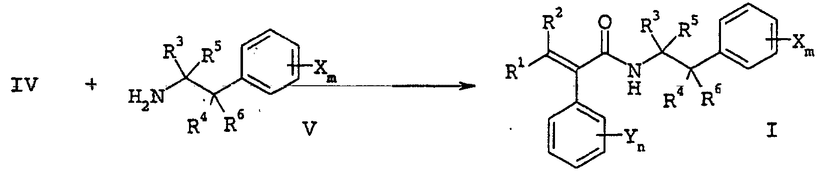

Die Edukte werden im allgemeinen in äquimolaren Mengen miteinander umgesetzt. Es kann für die Ausbeute vorteilhaft sein, V in einem Überschuß bezogen auf IV einzusetzen.

Alternativ können Carbonsäuren der Formel IV vor Amidierung mit V zunächst aktiviert werden, etwa durch überführung in Säurehalogenide, insbesondere in Säurechloride der Formel IVa.

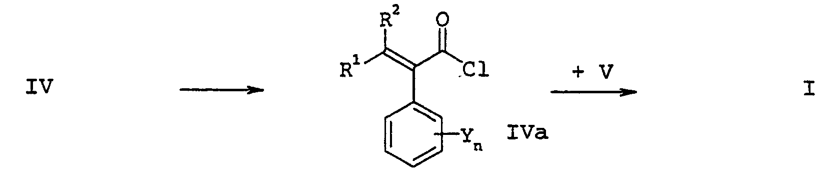

Chlorierung von Carbonsäuren IV erfolgt üblicherweise bei Temperaturen von -20°C bis 100°C, vorzugsweise -10°C bis 80°C, in einem inerten organischen Lösungsmittel [vgl. Organikum, 16. Aufl., S. 423ff., VEB Deutscher Verlag der Wissenschaften, Berlin 1985].

Als Chlorierungsmittel bei dieser Umsetzung eignen sich die üblichen anorganischen und organischen Chlorierungsmittel , z.B. Thionylchlorid, Oxalylchlorid, Phosphortrichlorid, Phosphorpentachlorid, Phosphoroxychlorid, Triphenylphosphin/CCl₄, vorzugsweise Thionylchlorid.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid, Dimethylformamid und Dimethylacetamid, besonders bevorzugt Acetonitril, Toluol und Tetrahydrofuran. Es können auch Gemische der genannten Lösungsmittel verwendet werden.

Die Chlorierungsmittel werden im allgemeinen mindestens in äquimolaren Mengen eingesetzt. Es kann für die Ausbeute vorteilhaft sein, sie in einem Überschuß von bis zu 10 mol bezogen auf 1 mol IV, vorzugsweise bis zu 5 mol, insbesondere bis zu 3 mol, einzusetzen.

Die Amidierung der Säurehalogenide mit dem Phenethylamin der Formel V erfolgt wie am Beispiel der Verbindung IVa illustriert:

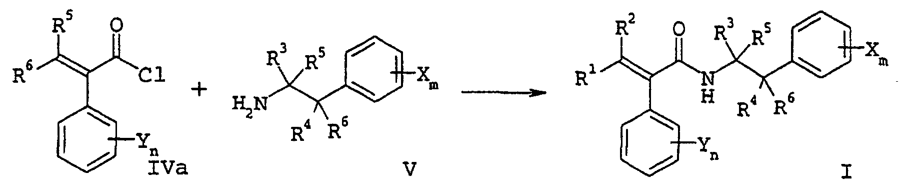

Diese Umsetzung erfolgt üblicherweise bei Temperaturen von 0°C bis 80°C, vorzugsweise 20°C bis 40°C, in einem inerten organischen Lösungsmittel in Gegenwart einer Base [vgl. Organikum, 16. Aufl., S. 412ff., VEB Deutscher Verlag der Wissenschaften, Berlin 1985].

Geeignete Lösungsmittel sind Ether wie Diethylether, Diisopropylether, tert.-Butylmethylether, Dioxan, Anisol und THF, Nitrile wie Acetonitril und Propionitril, sowie Dimethylsulfoxid, Dimethylformamid und Dimethylacetamid, besonders bevorzugt Diethylether und THF. Es können auch Gemische der genannten Lösungsmittel verwendet werden.

Als Basen kommen allgemein anorganische Verbindungen wie Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin und Pyridin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, V in einem Überschuß bezogen auf IVa einzusetzen.

Die für die Herstellung der Verbindungen I benötigten Ausgangsstoffe der Formel II sind in der Literatur bekannt, z. T. kommerziell erhältlich oder können auf folgenden Wegen hergestellt werden:

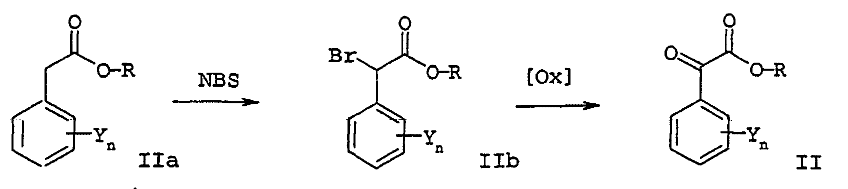

Die Bromierung mit N-Bromsuccinimid (NBS) oder 1,3-Dibrom-5,5-dimethylhydrantoin erfolgt üblicherweise bei Temperaturen von 0°C bis 200°C, vorzugsweise 20°C bis 110°C, in einem inerten organischen Lösungsmittel in Gegenwart eines Radikalstarters [vgl. Synthetic Reagents, Bd. 2, S. 1-63, Verlag Wiley, New York (1974); J. Heterocyclic Chem. S. 1431-1436 (1993); Synth. Commun. S. 2803ff. (1996); J. Med. Chem. S. 481ff. (1981)].

Bromverbindungen IIb werden direkt zu α-Ketoestern II oxidiert.
Die Oxidation mit N-Methylmorpholinoxid oder p-Dimethylaminopyridinoxid erfolgt üblicherweise bei Temperaturen von 0°C bis 100°C, vorzugsweise 20°C bis 60°C, in Dimethylsulfoxid [vgl.Bull. Chem. Soc. Jpn., S. 2221 (1981)].

Alternativ können Phenylessigsäureester IIa auch direkt zu α-Ketoestern II oxidiert werden. Die Oxidation kann beispielsweise mit SeO₂ oder KMnO₄ erfolgen, sie erfolgt üblicherweise bei Temperaturen von 20°C bis 180°C, vorzugsweise 20°C bis 120°C, in einem inerten organischen Lösungsmittel [vgl. Synthesis, S. 915 (1994; Synth Commun., S. 1253 (1988); J. Gen. Chem. USSR, Bd. 21, S. 694ff. (1951)].

Die für die Herstellung der Verbindungen II benötigten Phenylessigsäureester IIa sind in der Literatur bekannt, bzw. kommerziell erhältlich.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4 Kohlenstoffatomen, z.B. Methyl, Ethyl, Propyl, 1-Methylethyl, Butyl, 1-Methyl-propyl, 2-Methylpropyl, 1,1-Dimethylethyl;
**Halogenalkyl:** geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 8 Kohlenstoffatomen (wie vorstehend genannt), welche über ein Sauerstoffatom (-O-) an das Gerüst gebunden sind;
**Halogenalkoxy:** geradkettige oder verzweigte Halogenalkylgruppen mit 1 bis 4 Kohlenstoffatomen (wie vorstehend genannt), welche über ein Sauerstoffatom (-O-) an das Gerüst gebunden sind;

Im Hinblick auf ihre bestimmungsgemäße Verwendung der Phenethylacrylamide der Formel I sind die folgenden Bedeutungen der Substituenten, und zwar jeweils für sich allein oder in Kombination, besonders bevorzugt:

Die besonders bevorzugten Ausführungsformen der Zwischenprodukte in Bezug auf die Variablen entsprechen denen der Reste Xₘ, Yₙ, R¹, R², R³, R⁴, R⁵ und R⁶ der Formel I.

Insbesondere werden Verbindungen I bevorzugt, in denen R¹ und R² gleich sind und Chlor, Fluor oder Methyl bedeuten.

Daneben werden Verbindungen I besonders bevorzugt, in denen R¹ und R² für Chlor stehen.

Gleichermaßen besonders bevorzugt sind Verbindungen I, in denen R¹ und R² für Fluor stehen.

Desweiteren werden Verbindungen I besonders bevorzugt, in denen R¹ und R² für Methyl stehen.

Besonders bevorzugt sind auch Verbindungen der Formel I, in denen R¹ und R² verschieden sind, insbesondere R¹ einen voluminöseren Rest darstellt als R².

Gleichermaßen besonders bevorzugt sind Verbindungen der Formel I, in denen R² Wasserstoff bedeutet.

Besonders bevorzugt werden auch Verbindungen der Formel I, in denen Xₘ 3-C₁-C₄-Alkoxy, 4-O-C(R^{a},R^{b})-C≡C-R^{c} bedeutet. Diese Verbindungen entsprechen der Formel I'; X' bedeutet darin C₁-C₄-Alkoxy.

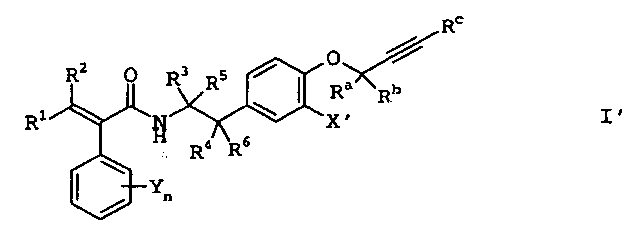

Daneben werden Verbindungen I besonders bevorzugt, in denen X C₁-C₈-Alkoxy und Y Halogen bedeutet

Desweiteren werden Verbindungen der Formel I besonders bevorzugt, in denen X für 3-Methoxy steht.

Besonders bevorzugt werden Verbindungen I, in denen R³ bis R⁶ für Wasserstoff stehen; diese Verbindungen werden durch die Formel I.1 beschrieben:

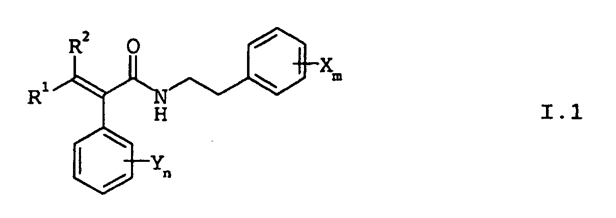

Außerdem werden Verbindungen I besonders bevorzugt, in denen R⁵ und R⁶ für Wasserstoff stehen; diese Verbindungen werden durch die Formel I.2 beschrieben:

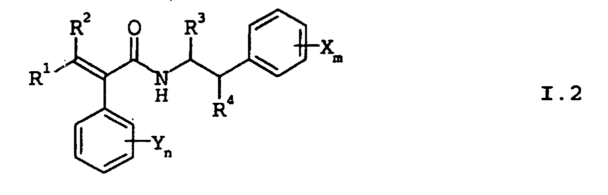

Gleichermaßen besonders bevorzugt sind Verbindungen der Formel I.2, in der R¹ und R² jeweils gleich sind und für Chlor, Fluor oder Methyl, R³ und R⁴ für Wasserstoff stehen oder gemeinsam einen Cyclopropylring bilden, Yₙ in der 4- oder 3,4-Position steht und Chlor, Fluor, Methyl oder Ethyl bedeutet, X für Chlor, Fluor, C₁-C₃-Alkyl, C₁-C₃-Alkoxy, Benzyloxy, Allyloxy, Propargyloxy, Trifluormethoxy oder Difluormethoxy steht und m 1 oder 2 bedeutet, wobei, wenn m 2 ist, X in der 3,4-Position steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel I.1, in denen R¹ und R² für Chlor und Yₙ für 4-Chlor steht und der Rest Xₘ für eine Verbindung jeweils einer Zeile der Tabelle A entspricht

### Tabelle 2

Verbindungen der Formel I.1, in denen R¹ und R² für Chlor und Yₙ für 3,4-Dichlor steht und der Rest Xₘ für eine Verbindung jeweils einer Zeile der Tabelle A entspricht einer Zeile der Tabelle A entspricht

### Tabelle 4

Verbindungen der Formel I.1, in denen R¹ und R² für Chlor und Yₙ für 4-Fluor steht und der Rest Xₘ für eine Verbindung jeweils einer Zeile der Tabelle A entspricht

### Tabelle 5

Verbindungen der Formel I.1, in denen R¹ und R² für Chlor und Yₙ für 3,4-Difluor steht und der Rest Xₘ für eine Verbindung jeweils einer Zeile der Tabelle A entspricht

### Tabelle 7

Verbindungen der Formel I.1, in denen R¹ und R² für Chlor und Yₙ für 4-CH₃ steht und der Rest Xₘ für eine Verbindung jeweils einer Zeile der Tabelle A entspricht

### Tabelle 8

Verbindungen der Formel I.1, in denen R¹ und R² für Chlor und Yₙ für 3,4-(CH₃)₂ steht und der Rest Xₘ für eine Verbindung jeweils einer Zeile der Tabelle A entspricht

### Tabelle 10

Verbindungen der Formel T.I, in denen R¹ und R² für Chlor und Yₙ für 4-CH₂CH₃ steht und der Rest Xₘ für eine Verbindung jeweils einer Zeile der Tabelle A entspricht

### Tabelle 11

Verbindungen der Formel I.1, in denen R¹ und R² für Chlor und Yₙ für 3,4-(CH₂CH₃)₂ steht und der Rest Xₘ für eine Verbindung jeweils einer Zeile der Tabelle A entspricht

### Tabelle 13

Verbindungen der Formel I.1, in denen R¹ und R² für Chlor und Yₙ für 4-CH(CH₃)₂ steht und der Rest Xₘ für eine Verbindung jeweils einer Zeile der Tabelle A entspricht

### Tabelle 14

Verbindungen der Formel I.1, in denen R¹ und R² für Chlor und Yₙ für 4-C(CH₃)₃ steht und der Rest Xₘ für eine Verbindung jeweils einer Zeile der Tabelle A entspricht

### Tabelle 15

Verbindungen der Formel I.1, in denen R¹ und R² für Chlor und Yₙ für 4-CF₃ steht und der Rest Xₘ für eine Verbindung jeweils einer Zeile der Tabelle A entspricht

### Tabelle 16

Verbindungen der Formel I.1, in denen R¹ und R² für Chlor und Yₙ für 3,4-(CF₃)₂ steht und der Rest Xₘ für eine Verbindung jeweils einer Zeile der Tabelle A entspricht

### Tabelle 18

Verbindungen der Formel I.1, in denen R¹ und R² für Chlor und Yₙ für 4-OCH₃ steht und der Rest Xₘ für eine Verbindung jeweils einer Zeile der Tabelle A entspricht

### Tabelle 19

Verbindungen der Formel I.1, in denen R¹ und R² für Chlor und Yₙ für 3,4-(OCH₃)₂ steht und der Rest Xₘ für eine Verbindung jeweils einer Zeile der Tabelle A entspricht

### Tabelle 21

Verbindungen der Formel I.1, in denen R¹ und R² für Chlor und Yₙ für 4-OCF₃ steht und der Rest Xₘ für eine Verbindung jeweils einer Zeile der Tabelle A entspricht

### Tabelle 22

Verbindungen der Formel I.1, in denen R¹ und R² für Chlor und Yₙ für 4-OCHF₂ steht und der Rest Xₘ für eine Verbindung jeweils einer Zeile der Tabelle A entspricht

### Tabelle 23

Verbindungen der Formel I.1, in denen R¹ und R² für Chlor und Yₙ für 4-Phenyl steht und der Rest Xₘ für eine Verbindung jeweils einer Zeile der Tabelle A entspricht

### Tabelle 24

Verbindungen der Formel I.1, in denen R¹ und R² für Fluor und Yₙ für 4-Chlor steht und der Rest Xₘ für eine Verbindung jeweils einer Zeile der Tabelle A entspricht

### Tabelle 25

Verbindungen der Formel I.1, in denen R¹ und R² für Fluor und Yₙ für 3,4-Dichlor steht und der Rest Xₘ für eine Verbindung jeweils einer Zeile der Tabelle A entspricht

### Tabelle 27

Verbindungen der Formel I.1, in denen R¹ und R² für Fluor und Yₙ für 4-Fluor steht und der Rest Xₘ für eine Verbindung jeweils einer Zeile der Tabelle A entspricht

### Tabelle 28

Verbindungen der Formel I.1, in denen R¹ und R² für Fluor und Yₙ für 3,4-Difluor steht und der Rest Xₘ für eine Verbindung jeweils einer Zeile der Tabelle A entspricht

### Tabelle 30

Verbindungen der Formel I.1, in denen R¹ und R² für Fluor und Yₙ für 4-CH₃ steht und der Rest Xₘ für eine Verbindung jeweils einer Zeile der Tabelle A entspricht

### Tabelle 31

Verbindungen der Formel I.1, in denen R¹ und R² für Fluor und Yₙ für 3,4-(CH₃)₂ steht und der Rest Xₘ für eine Verbindung jeweils einer Zeile der Tabelle A entspricht

### Tabelle 33

Verbindungen der Formel I.1, in denen R¹ und R² für Methyl und Yₙ für 4-Chlor steht und der Rest Xₘ für eine Verbindung jeweils einer Zeile der Tabelle A entspricht

### Tabelle 34

Verbindungen der Formel I.1, in denen R¹ und R² für Methyl und Yₙ für 3,4-Dichlor steht und der Rest Xₘ für eine Verbindung jeweils einer Zeile der Tabelle A entspricht

### Tabelle 36

Verbindungen der Formel I.1, in denen R¹ und R² für Methyl und Yₙ für 4-Fluor steht und der Rest Xₘ für eine Verbindung jeweils einer Zeile der Tabelle A entspricht

### Tabelle 37

Verbindungen der Formel I.1, in denen R¹ und R² für Methyl und Yₙ für 3,4-Difluor steht und der Rest Xₘ für eine Verbindung jeweils einer Zeile der Tabelle A entspricht

### Tabelle 39

Verbindungen der Formel I.1, in denen R¹ und R² für Methyl und Yₙ für 4-CH₃ steht und der Rest Xₘ für eine Verbindung jeweils einer Zeile der Tabelle A entspricht

### Tabelle 40

Verbindungen der Formel I.1, in denen R¹ und R² für Methyl und Yₙ für 3,4-(CH₃)₂ steht und der Rest Xₘ für eine Verbindung jeweils einer Zeile der Tabelle A entspricht

### Tabelle 42

Verbindungen der Formel I.1, in denen R¹ für Methyl, R² für Wasserstoff und Yₙ für 4-Chlor steht und der Rest Xₘ für eine Verbindung jeweils einer Zeile der Tabelle A entspricht

### Tabelle 43

Verbindungen der Formel I.1, in denen R¹ für Methyl, R² für Wasserstoff und Yₙ für 3,4-Dichlor steht und der Rest Xₘ für eine Verbindung jeweils einer Zeile der Tabelle A entspricht

### Tabelle 45

Verbindungen der Formel I.1, in denen R¹ für Methyl, R² für Wasserstoff und Yₙ für 4-Fluor steht und der Rest Xₘ für eine Verbindung jeweils einer Zeile der Tabelle A entspricht

### Tabelle 46

Verbindungen der Formel I.1, in denen R¹ für Methyl, R² für Wasserstoff und Yₙ für 3,4-Difluor steht und der Rest Xₘ für eine Verbindung jeweils einer Zeile der Tabelle A entspricht

### Tabelle 48

Verbindungen der Formel I.1, in denen R¹ für Methyl, R² für Wasserstoff und Yₙ für 4-CH₃ steht und der Rest Xₘ für eine Verbindung jeweils einer Zeile der Tabelle A entspricht

### Tabelle 49

Verbindungen der Formel I.1, in denen R¹ für Methyl, R² für Wasserstoff und Yₙ für 3,4-(CH₃)₂ steht und der Rest Xₘ für eine Verbindung jeweils einer Zeile der Tabelle A entspricht

### Tabelle 51

Verbindungen der Formel I.1, in denen R¹ für Ethyl, R² für Wasserstoff und Yₙ für 4-Chlor steht und der Rest Xₘ für eine Verbindung jeweils einer Zeile der Tabelle A entspricht

### Tabelle 52

Verbindungen der Formel I.1, in denen R¹ für Ethyl, R² für Wasserstoff und Yₙ für 3,4-Dichlor steht und der Rest Xₘ für eine Verbindung jeweils einer Zeile der Tabelle A entspricht

### Tabelle 54

Verbindungen der Formel I.1, in denen R¹ für Ethyl, R² für Wasserstoff und Yₙ für 4-Fluor steht und der Rest Xₘ für eine Verbindung jeweils einer Zeile der Tabelle A entspricht

### Tabelle 55

Verbindungen der Formel I.1, in denen R¹ für Ethyl, R² für Wasserstoff und Yₙ für 3,4-Difluor steht und der Rest Xₘ für eine Verbindung jeweils einer Zeile der Tabelle A entspricht

### Tabelle 57

Verbindungen der Formel I.1, in denen R¹ für Ethyl, R² für Wasserstoff und Yₙ für 4-CH₃ steht und der Rest Xₘ für eine Verbindung jeweils einer Zeile der Tabelle A entspricht

### Tabelle 58

Verbindungen der Formel I.1, in denen R¹ für Ethyl, R² für Wasserstoff und Yₙ für 3,4-(CH₃)₂ steht und der Rest Xₘ für eine Verbindung jeweils einer Zeile der Tabelle A entspricht

### Tabelle 60

Verbindungen der Formel I.2, in denen R¹ und R² für Chlor und Yₙ für 4-Chlor steht, R³ und R⁴ einen Cyclopropylring bilden und der Rest Xₘ für eine Verbindung jeweils einer Zeile der Tabelle A entspricht

### Tabelle 61

Verbindungen der Formel I.2, in denen R¹ und R² für Fluor und Yₙ für 4-Chlor steht, R³ und R⁴ einen Cyclopropylring bilden und der Rest Xₘ für eine Verbindung jeweils einer Zeile der Tabelle A entspricht

### Tabelle 62

Verbindungen der Formel I.2, in denen R¹ und R² für Methyl und Yₙ für 4-Chlor steht, R³ und R⁴ einen Cyclopropylring bilden und der Rest Xₘ für eine Verbindung jeweils einer Zeile der Tabelle A entspricht

### Tabelle 63

Verbindungen der Formel I.2, in denen R¹ für Methyl, R² für Wasserstoff und Yₙ für 4-Chlor steht R³ und R⁴ einen Cyclopropylring bilden und der Rest Xₘ für eine Verbindung jeweils einer Zeile der Tabelle A entspricht

### Tabelle 64

Verbindungen der Formel I.2, in denen R¹ für Ethyl, R² für Wasserstoff und Yₙ für 4-Chlor steht R³ und R⁴ einen Cyclopropylring bilden und der Rest Xₘ für eine Verbindung jeweils einer Zeile der Tabelle A entspricht

**Tabelle A**

| **Nr**. | **X**_{**m**} |
|---|---|
| A-1 | 2-Cl |
| A-2 | 3-Cl |
| A-3 | 4-Cl |
| A-4 | 2-F |
| A-5 | 3-F |
| A-6 | 4-F |
| A-7 | 2-CH₃ |
| A-8 | 3-CH₃ |
| A-9 | 4-CH₃ |
| A-10 | 2-CH₂CH₃ |
| A-11 | 3-CH₂CH₃ |
| A-12 | 4-CH₂CH₃ |
| A-13 | 3-CH₂CH₂CH₃ |
| A-14 | 4-CH₂CH₂CH₃ |
| A-15 | 3-CH(CH₃)₂ |
| A-16 | 4-CH(CH₃)₂ |
| A-17 | 2-OCH₃ |
| A-18 | 3-OCH₃ |
| A-19 | 4-OCH₃ |
| A-20 | 2-OCH₂CH₃ |
| A-21 | 3-OCH₂CH₃ |
| A-22 | 4-OCH₂CH₃ |
| A-2 3 | 3-OCH₂CH₂CH₃ |
| A-24 | 4-OCH₂CH₂CH₃ |
| A-25 | 3-OCH(CH₃)₂ |
| A-26 | 4-OCH(CH₃)₂ |
| A-27 | 2,3-(CH₃)₂ |
| A-28 | 3,4-(CH₃)₂ |
| A-29 | 3,5-(CH₃)₂ |
| A-30 | 2,6-(CH₃)₂ |
| A-31 | 2,3-(CH₂CH₃)₂ |
| A-32 | 3,4-(CH₂CH₃)₂ |
| A-33 | 3,5-(CH₂CH₃)₂ |
| A-34 | 2,6-(CH₂CH₃)₂ |
| A-35 | 3-CH₃, 4-CH₂CH₃ |
| A-36 | 3-CH₂CH₃, 4-CH₃ |
| A-37 | 3-CH₃, 5-CH₂CH₃ |
| A-38 | 2-CH₂CH₃, 6-CH₃ |
| A-39 | 2,3-(OCH₃)₂ |
| A-40 | 3,4-(OCH₃)₂ |
| A-41 | 3,5-(OCH₃)₂ |
| A-42 | 2,6-(OCH₃)₂ |
| A-43 | 2,3-(OCH₂CH₃)₂ |
| A-44 | 3,4-(OCH₂CH₃)₂ |
| A-45 | 3,5-(OCH₂CH₃)₂ |
| A-46 | 2,6-(OCH₂CH₃)₂ |
| A-47 | 3-OCH₃, 4-OCH₂CH₃ |
| A-48 | 3-OCH₂CH₃, 4-OCH₃ |
| A-49 | 3-OCH₃, 5-OCH₂CH₃ |
| A-50 | 2-OCH₂CH₃, 6-OCH₃ |
| A-51 | 3-OCH₃, 4-OCH₂CH₂CH₃ |
| A-52 | 3-OCH₂CH₂CH₃, 4-OCH₃ |
| A-53 | 3-OCH₂CH₃, 4-OCH₂CH₂CH₃ |
| A-54 | 3-OCH₂CH₂CH₃, 4-OCH₂CH₃ |
| A-55 | 3-OCH₃, 4-OCH(CH₃)₂ |
| A-56 | 3-OCH(CH₃)₂, 4-OCH₃ |
| A-57 | 3-OCH₃, 4-OCH₂-C₆H₅ |
| A-58 | 3-OCH₂-C₆H₅, 4-OCH₃ |
| A-59 | 3,4-(OCH₂-C₆H₅)₂ |
| A-60 | 3-OCH₃, 4-OCH₂-CH=CH₂ |
| A-61 | 3-OCH₃, 4-OCH₂-C≡CH |
| A-62 | 3-OCH₃, 4-OCH(CH₃)-C≡CH |
| A-63 | 3-OCH₃, 4-OCH(CH₃)-C≡C-CH₃ |
| A-64 | 3-OCH₃, 4-OCH(CH₃)-C≡C-CH₂CH₃ |
| A-65 | 3-OCH₃, 4-OC(CH₃)₂-C≡CH |
| A-66 | 3-OCH₃, 4-OC(CH₃)₂-C≡C-CH₃ |
| A-67 | 3-OCH₃, 4-OC(CH₃)₂-C≡C-CH₂CH₃ |
| A-68 | 3-OCH₃, 4-OCH₂-C≡C-C₆H₅ |
| A-69 | 3-OCH₃, 4-OCH(CH₃)-C≡C-C₆H₅ |
| A-70 | 3-OCH₃, 4-OC(CH₃)₂-C≡C-C₆H₅ |
| A-71 | 3-OCH₃, 4-OCH₂-C≡C-(4-Cl-C₆H₄) |
| A-72 | 3-OCH₃, 4-OCH(CH₃)-C≡C-(4-Cl-C₆H₄) |
| A-73 | 3-OCH₃, 4-OC(CH₃)₂-C≡C-(4-Cl-C₆H₄) |
| A-74 | 3-OCH₃, 4-OCF₃ |
| A-75 | 3-OCH₃, 4-OCHF₂ |

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.
Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und.Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
- I.: 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
- II.: 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
- III.: 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
- IV.: 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
- V.: 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
- VI.: Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
- VII.: 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
- VIII.: 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Lignin-sulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volumeverfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-ylethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl-ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1: Herstellung von 2-Brom-(4-chlorphenyl)-essigsäuremethylester

Zu der Lösung von 215,3 g (1,17 mol) 4-Chlor-phenylessigsäuremethylester in 500 ml Tetrachlormethan gab man portionsweise 116 g (0,585 mol) 1,3-Dibrom-5,5-dimethylhyntantoin sowie 1 g AIBN und refluxierte 24 Stunden. Anschließend wurde mit Wasser und 1 N Natronlauge gewaschen, die Wasserphasen mit Methylenchlorid extrahiert und die organischen Phasen getrocknet und eingeengt. Man erhielt 260 g der Titelverbindung.
¹H-NMR [δ, (CDCl₃)]: 3,8 (s,3H); 5,3 (s,1H).

### Beispiel 2: Hestellung von (4-Chlorphenyl)-2-oxo-essigsäuremethylester

Eine Lösung von 100 g (0,38 mol) der Verbindung aus Beispiel 1 in 400 ml Dimethylsulfoxid (DMSO) wurde unter Eiskühlung mit 97,2 g 75%iger wässriger N-Methylmorpholin-N-oxid-Lösung versetzt. Nach 24 Stunden Rühren bei 20 bis 25 °C wurde auf Wasser gegossen und mit Methyl-tert.Butylether (MtBE) extrahiert. Aus den organischen Phasen wurde nach Trocknung und Abdestillieren des Lösungsmittels und Chromatographie an Kieselgel (Cyclohexan/MtBE [3:1]) 69 g der Titelverbindung erhalten.
¹H-NMR [δ, (CDCl₃)]: 4,0 (s,3H); 7,5 (m, 2H) und 8,0 (m,2H).

### Beispiel 3: Herstellung von 3,3-Dichlor-2-(4-chlorphenyl)-acrylsäuremethylester

Zu einer Lösung von 29,9 g (0,15 mol) des Ketoesters aus Beispiel 2 in 300 ml Acetonitril wurde nach Versetzen mit 117,9 g PPh₃ (0,45 mol) 69,3 g (0,45 mol) Tetrachlorkohlenstoff zugetropft. Nach 4 Std. bei etwa 56 °C wurde die Reaktionslösung eingeengt; der erhaltene Rückstand über Kieselgel mit Cyclohexan/MtBE (3:1) chromatographiert. Man erhielt 39,2 g der Titelverbindung.
¹H-NMR [δ, (CDCl₃)]: 3,8 (s, 3H); 7,3-7,4 (m,4H).

### Beispiel 4: Herstellung von 3,3-Dichlor-2-(4-chlorphenyl)-acrylsäure

Eine Lösung von 17,1 g (64 mmol) des Esters aus Beispiel 3 in 50 ml Methanol wurde nach Versetzen mit 193 ml 1 N KOH-Lösung für 20 Std. bei 20 bis 25 °C gerührt. Nach Abdestillieren des Lösungsmittels wurde der Rückstand mit 20%iger H₂SO₄ auf pH 1 angesäuert und mit Essigester extrahiert. Aus den organischen Phasen wurden nach Trocknung und Abdestillieren des Lösungsmittels 15 g der Titelverbindung isoliert.
¹H-NMR [δ, (CDCl₃)]: 7,25 (m, 2H); 7,40 (m,2H) und 9,5 (s,1H).

### Beispiel 5: Herstellung von 3,3-Dichlor-2-(4-chlorphenyl)-acrylsäurechlorid

Zu der Lösung von 50 g (0,199 mol) der Säure aus Beispiel 4 in 200 ml Diethylether und 23,6 g (0,298 mol) Pyridin tropfte man bei 0 °C 35,5 g (0,298 mol) Thionylchlorid. Die Lösung wurde für 6 Std. bei 20 bis 25 °C gerührt, dann filtriert. Nach Abdestillieren des Lösungsmittels erhielt man 42,1 g des Produktes, das ohne Reinigung weiter umgesetzt wurde.

### Beispiel 6: Herstellung von 3,3-Dichlor-2-(4-chlorphenyl)-N-[2-(3,4-dimethoxyphenyl)-ethyl]-acrylamid [I-1]

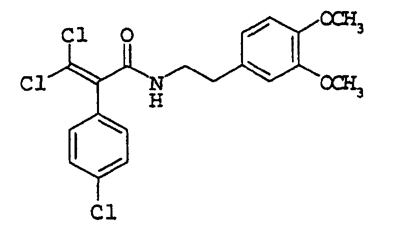

Zu einer Lösung von 7,24 g (0,04 mol) Homoveratrylamin und 4,04 g (0,04 mol) Triethylamin in 80 ml Methylenchlorid tropfte man eine Lösung von 10,8 g (0,04 mol) des in Beispiel 5 hergestellten Säurechlorides in 20 ml Methylenchlorid. Die Lösung wurde 3 Std. bei 20 bis 25 °C gerührt; mit 3N Natronlauge und dann mit 10 Gew.-%iger Salzsäure gewaschen. Die organische Phase wurde vom Lösungsmittel befreit, aus dem Rückstand erhielt man nach Chromatographie an Kieselgel mit Cyclohehan/MtBE (3:1) 13,85 g der Titelverbindung.
¹H-NMR [δ, (CDCl₃)]: 2,8 (m, 2H); 3,55 (m,2H); 3,8 (s,3H); 3,85 (s,3H); 5,6 (s, 1H); 6,60-6,80 (m, 3H); 7,25-7,40 (m, 4H).

### Beispiel 7: Herstellung von 4-(2-Aminoethyl)-2-methoxyphenol

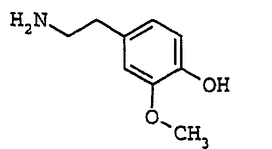

5 g (19,5 mmol) 2-[4-(Benzyloxy)-3-methoxyphenyl]ethylamin [vgl.: Heterocycles, Vol. 28, 297-298 (1989)] in 100 ml Tetrahydrofuran wurden nach Zugabe von 0,5 g Palladium auf Aktivkohle (10%ig) bei Normaldruck hydriert. Nach Beendigung der Wasserstoffaufnahme wurde abfiltriert und das Filtrat vom Lösungsmittel befreit. Der Rückstand wurde mit Diisopropylether digeriert, abfiltriert und getrocknet.

IR (cm⁻¹): 2935, 1593, 1515, 1263, 1230, 1032.

### Beispiel 8: Herstellung von 3,3-Dichlor-2-(4-chlorophenyl)-N-[2-(4-hydroxy-3-methoxyphenyl)-ethyl]acrylamid-4-(2-aminoethyl)-2-methoxyphenol [I-52]

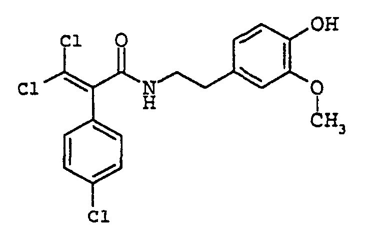

Zu 1,25 g 3,3-Dichlor-2-(4-chlorphenyl)-acrylsäurechlorid in 30 ml Dimethylformamid (DMF) gab man 1 g 4-(2-Aminoethyl)-2-methoxyphenol, 2,21 g 1-Benzotriazolyloxy-tris(dimethylamino)phosphoniumhexafluorophosphat (BOP) sowie 1,5 g N-Ethyl-diisopropylamin und rührte etwa 12 Std. bei 23°C nach. Dann wurde mit Wasser versetzt, mit Essigester extrahiert, die Extrakte mit NaCl-Lösung gewaschen und getrocknet. Nach Entfernen des Lösungsmittels wurde der Rückstand über Kieselgel mit Cyclohexan/Methyl-tert.Butyl-Ether (MtBE) (3:1) chromatographiert. Man erhielt die Titelverbindung in Form eines Öls.
MS (m/e): M+H 400, 368, 366, 360.

### Beispiel 9: Herstellung von 3,3-Dichlor-2-(4-chlorophenyl)-N-[2-(4-hydroxy-3-methoxyphenyl)ethyl]-acrylamid [I-53]

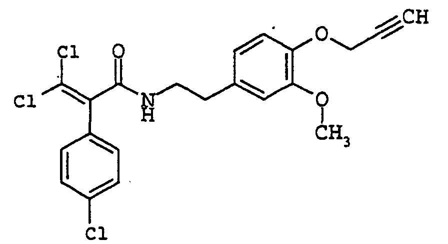

Eine Lösung von 0,4 g 3,3-Dichlor-2-(4-chlorophenyl)-N-[2-(4-hydroxy-3-methoxyphenyl)-ethyl]acrylamid in 50 ml DMF wurde nach. Zugabe von 0,14 g Kaliumcarbonat und 0,12 g Propargylbromid etwa 12 Std. bei 23°C gerührt. Nach Zugabe weiterer 0,14 g Kaliumcarbonat sowie 0,12 g Propargylbromid und weiteren 6 Std. Rühren bei 23°C wurde auf Wasser gegeben, mit MtBE extrahiert und die Extrakte mit Wasser gewaschen und getrocknet. Nach Entfernen des Lösungsmittels und Chromatographie an Kieselgel [Cyclohexan/MtBE (1:1)] erhielt man die Titelverbindung in Form eines Öls.
¹H-NMR (ppm): 2,5 (m, 1H); 2,8 (t, 2H); 3,6 (m, 2H); 3,8 (s, 3H); 4,78 (s, 2H).

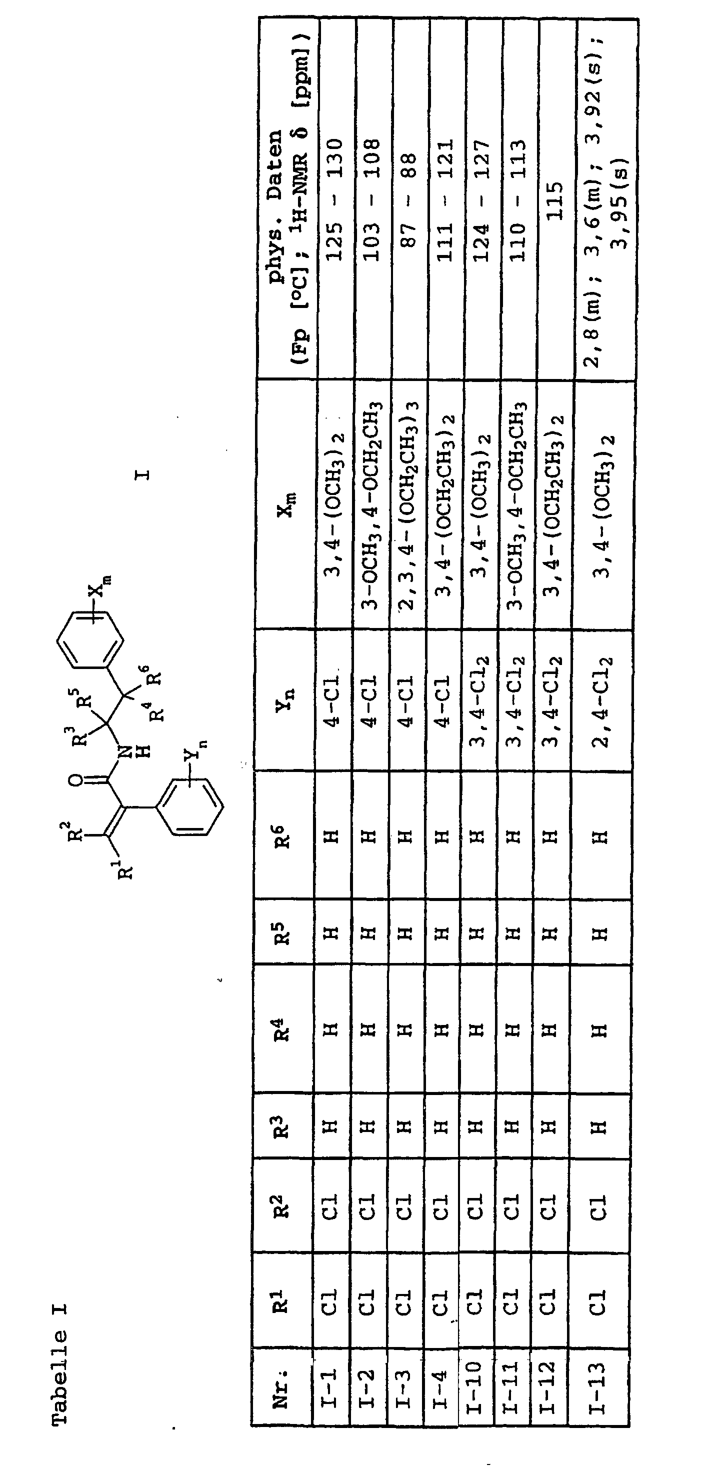

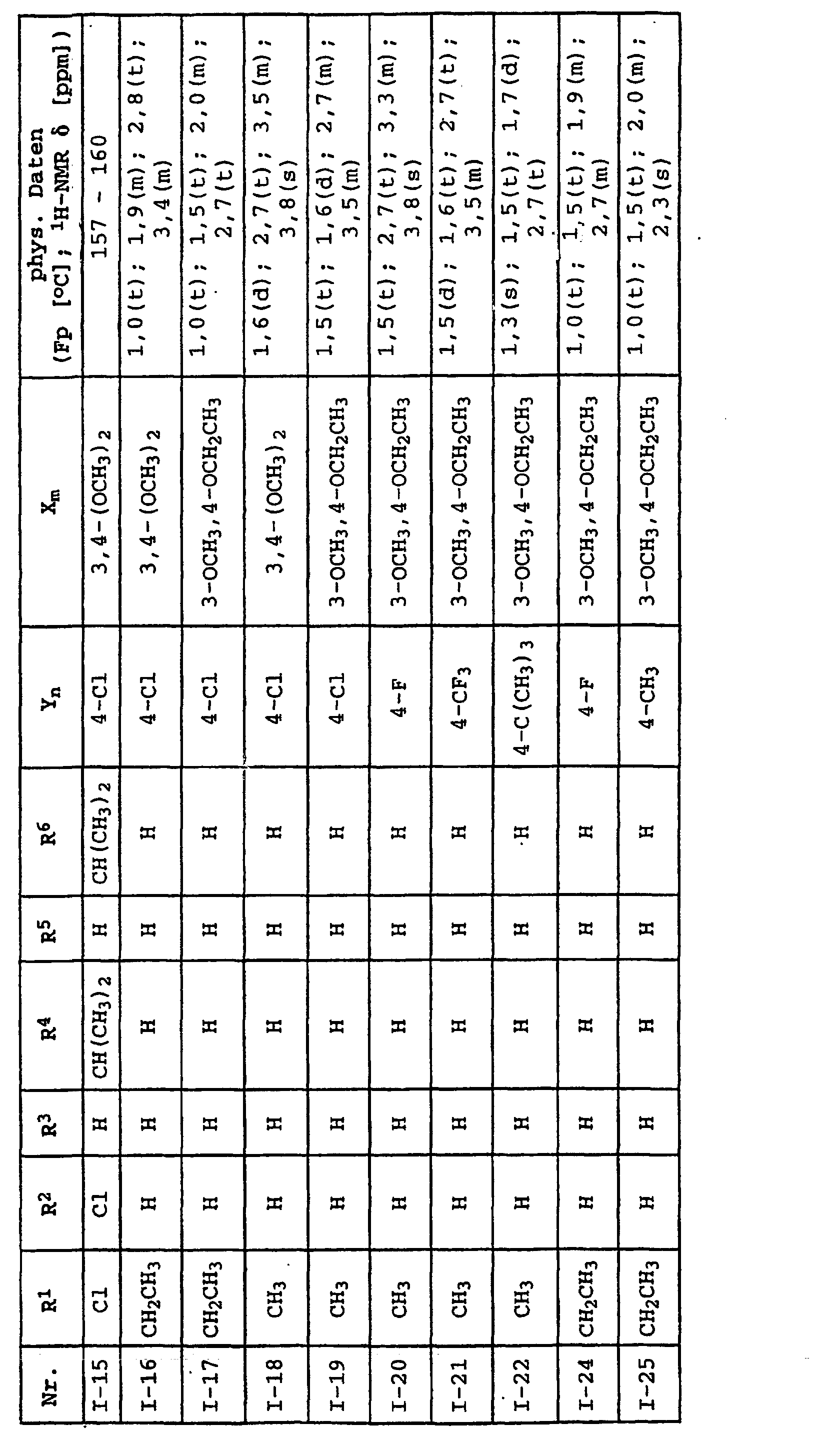

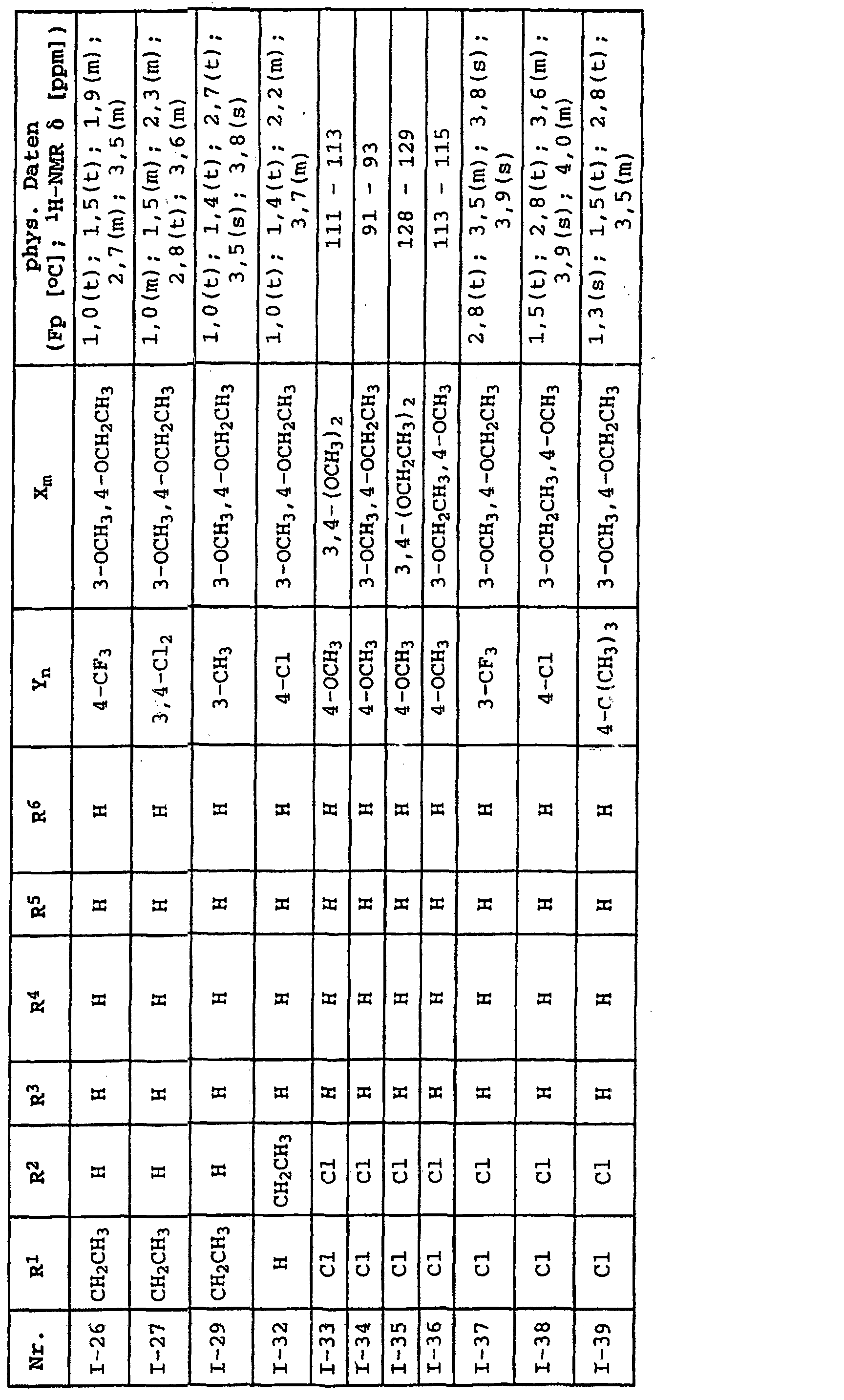

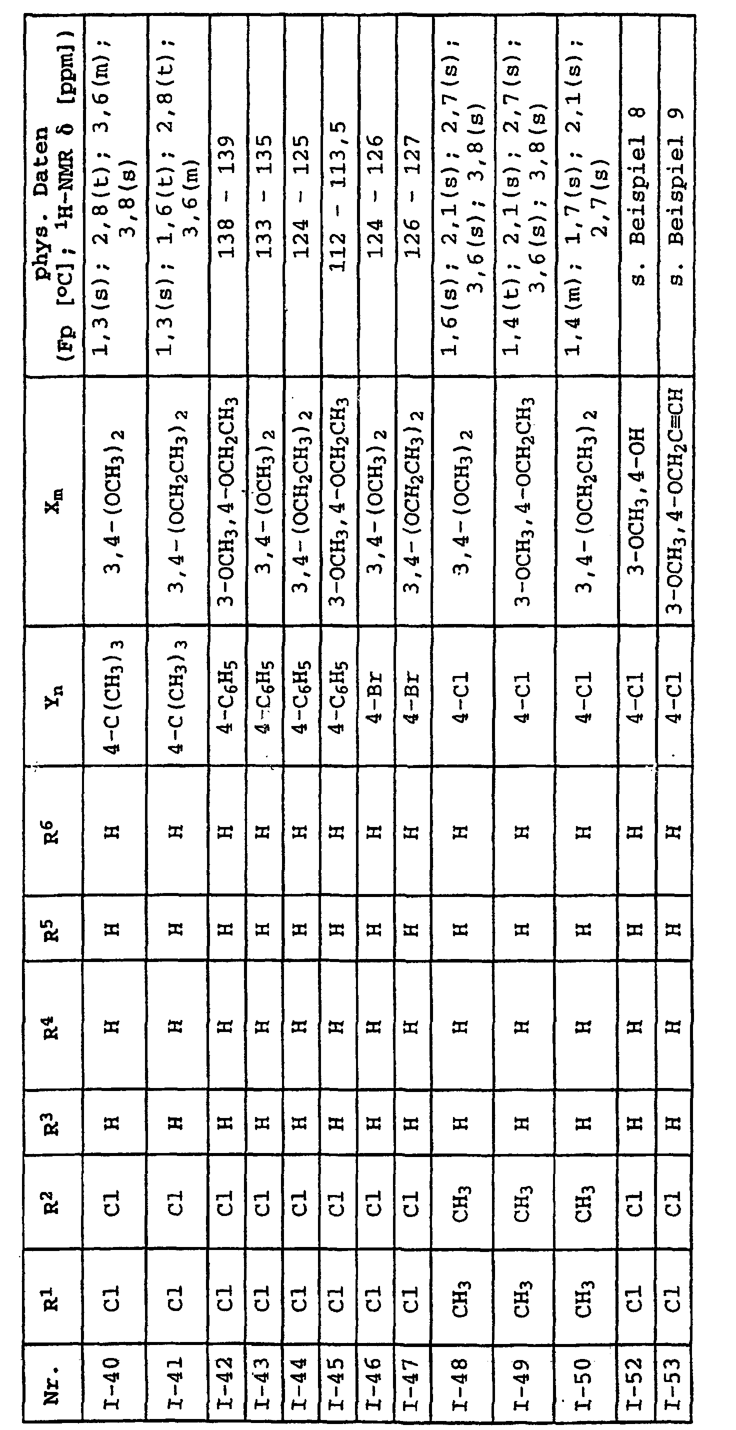

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

### Anwendungsbeispiel 1 - Wirksamkeit gegen Phytophthora infestans an Tomaten

Blätter von Topfpflanzen der Sorte "Große Fleischtomate St. Pierre" wurden mit einer wäßrigen Suspension, die aus einer Stammlösung aus 10 % Wirkstoff, 63 % Cyclohexanon und 27 % Emulgiermittel angesetzt wurde, bis zur Tropfnässe besprüht. Am folgenden Tag wurden die Blätter mit einer kalten wäßrigen Zoosporenaufschwemmung von Phytophthora infestans mit einer Dichte von 0.25 x 10⁶ Sporen/ml infiziert. Anschließend wurden die Pflanzen in einer wasserdampfgesättigten Kammer bei Temperaturen zwischen 18 und 20°C aufgestellt. Nach 6 Tagen hatte sich die Krautfäule auf den unbehandelten, jedoch infizierten Kontrollpflanzen so stark entwickelt, daß der Befall visuell in % ermittelt werden konnte.

In diesem Test zeigten die mit 250 ppm der Wirkstoffe I-1, I-2, I-11, I-17, I-19, I-25, I-26, I-27, I-32, I-33, I-34, I-39, I-40, I-42, I-43, I-45, I-46 und I-49 der Tabelle I behandelten Pflanzen nicht über 10 % Befall, während die unbehandelten Pflanzen zu 90 % befallen waren.

### Anwendungsbeispiel 2 - Wirksamkeit gegen Plasmopara viticola

Blätter von Topfreben der Sorte "Müller-Thurgau" wurden mit wäßriger Wirkstoffaufbereitung, die mit einer Stammlösung aus 10 % Wirkstoff, 63 % Cyclohexanon und 27 % Emulgiermittel angesetzt wurde, bis zur Tropfnässe besprüht. Am folgenden Tag wurden die Blätter mit einer wäßrigen Zoosporenaufschwemmung von Plasmopara viticola inokuliert. Danach wurden die Reben zunächst für 48 Stunden in einer wasserdampfgesättigten Kammer bei 24°C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

In diesem Test zeigten die mit 250 ppm der Wirkstoffe I-1, I-2, I-10, I-11, I-12, I-16, I-17, I-19, I-21, I22, I24 bis I-27, I-29, I-32, I-33, I-34, I-39, I-40, I-42, I-43, I-45, I-46, I-48 und I-49 der Tabelle I behandelten Pflanzen nicht über 10 % Befall, während die unbehandelten Pflanzen zu 90 % befallen waren.

### Vergleichsversuch - Dauerwirksamkeit gegen Phytophthora infestans an Tomaten

Getopfte Tomatenpflanzen der Sorte "Große Fleischtomate St. Pierre " wurden im 4 - Blattstadium mit einer wäßrigen Suspension, die aus einer Stammlösung aus 10 % Wirkstoff, 63 % Cyclohexanon und 27 % Emulgiermittel angesetzt wurde, bis zur Tropfnässe besprüht. Um die Dauerwirksamkeit der Verbindungen auszuprüfen, wurden die Blätter eine Woche nach der Applikation mit einer wäßrigen Zoosporenaufschwemmung von Phytophthora infestans infiziert. Anschließend wurden die Pflanzen in einer wasserdampfgesättigten Kammer bei Temperaturen zwischen 16 und 18°C aufgestellt. Nach 6 Tagen hatte sich die Krautfäule auf den unbehandelten, jedoch infizierten Kontrollpflanzen so stark entwickelt, daß der Befall visuell in % ermittelt werden konnte.

Als Vergleichswirkstoff diente die aus WO-A 96/23763 bekannte Verbindung der folgenden Struktur:

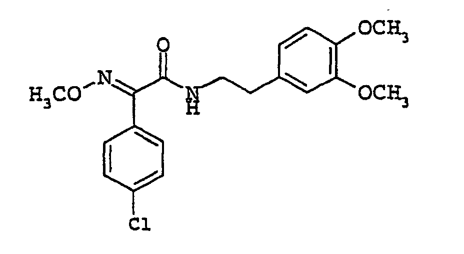

In diesem Test zeigten die mit 63 ppm der Wirkstoffe I-1, I-2, I-11, I-33 und I-34 der Tabelle I behandelten Pflanzen Null bis 25 % Befall, während die mit 63 ppm des Vergleichswirkstoffs behandelten Pflanzen zu 40 % befallen und die unbehandelten Pflanzen zu 100 % befallen waren.

## Patentansprüche

1. Verwendung von Phenethylacrylamiden der Formel I 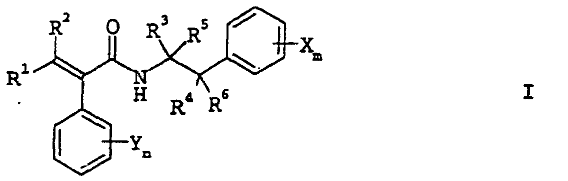 in der die Substituenten folgende Bedeutungen haben:
X Halogen, C₁-C₄-Alkyl, C₁-C₄-Halogenalkyl, C₁-C₈-Alkoxy, C₁-C₄-Halogenalkoxy und -O-C(R^{a},R^{b})-C≡C-R^{c};
R^{a},R^{b} unabhängig voneinander Wasserstoff und C₁-C₆-Alkyl;
R^{c} Wasserstoff, C₁-C₈-Alkyl, C₃-C₈-Cycloalkyl und Phenyl, welches substituiert sein kann durch Halogen, Cyano, Nitro, CF₃, C₁-C₄-Alkyl und/oder C₁-C₄-Alkoxy;
m 1 bis 4, wobei die Reste X verschieden sein können, wenn m größer als 1 ist;
n 1 oder 2, wobei die Reste Y verschieden sein können, wenn n=2 ist;
Y Halogen, Nitro, Cyano, C₁-C₄-Alkyl, CF₃, C₁-C₄-Alkoxy und Phenyl, wobei Y in 3-, 4- oder 3,4-Position steht;
R¹, R² unabhängig voneinander Wasserstoff, Halogen, C₁-C₄-Alkyl und CF₃;
R³,R⁴,R⁵,R⁶ unabhängig voneinander Wasserstoff, C₁-C₄-Alkyl und C₁-C₄-Alkoxy oder
R³ und R⁴ gemeinsam einen Cyclopropylring bilden, wobei die C-R⁵- und C-R⁶-Bindungen zueinander E- oder Z-ständig sein können;
zur Bekämpfung von pflanzenpathogenen Schadpilzen.

2. Phenethylacrylamide der Formel I nach Anspruch 1, in der R¹ und R² gleich sind und Cl, F und CH₃ bedeuten.

3. Phenethylacrylamide der Formel I nach Ansprüchen 1 oder 2, in der m 1 oder 2 bedeutet und X in 3-, 4- oder 3,4-Position steht.

4. Phenethylacrylamide der Formel I nach Ansprüchen 1 bis 3, in der X C₁-C₈-Alkoxy und Y Halogen bedeutet.

5. Phenethylacrylamide der Formel I nach Ansprüchen 1 bis 4, in der R³ und R⁴ Wasserstoff bedeuten.

6. Phenethylacrylamide der Formeln

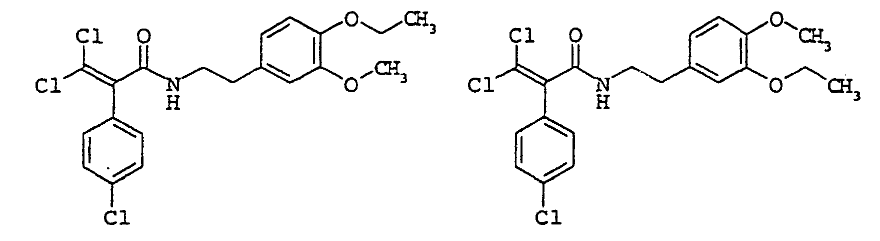

7. Verfahren zur Herstellung der Verbindungen der Ansprüche 2 bis 6, **dadurch gekennzeichnet, daß** man α-Ketoester der Formel II, 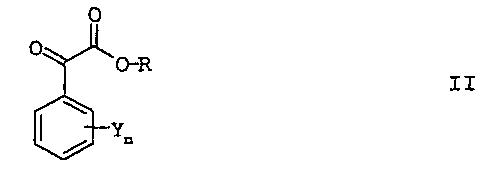 in der R C₁-C₄-Alkyl bedeutet, mit Triphenylphosphin und, wenn R¹ und R² Chlor bedeutet, mit CCl₄, oder, wenn R¹ und R² Fluor bedeutet, mit Natrium-Difluorchloracetat, oder, wenn R¹ und R² Methyl bedeutet, unter basischen Bedingungen mit iso-Propyltriphenylphosphoniumhalogenid, zu Acrylestern der Formel III, 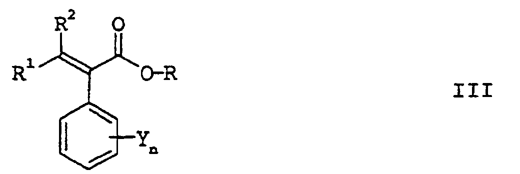 und III zu Carbonsäuren der Formel IV 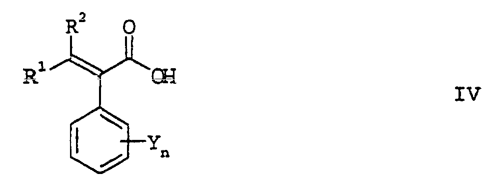 verseift und IV mit Phenethylaminen der Formel V 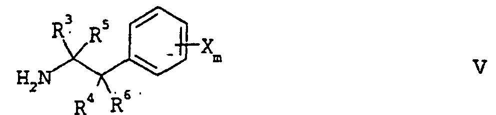 zu Verbindungen der Formel I kondensiert.

8. Zur Bekämpfung von pflanzenpathogenen Schadpilzen geeignetes Mittel, enthaltend einen festen oder flüssigen Trägerstoff und eine Verbindung der Formel I gemäß Ansprüchen 2 bis 6.

9. Verwendung der Verbindungen I gemäß Ansprüchen 2 bis 6 zur Herstellung eines zur Bekämpfung von pflanzenpathogenen Schadpilzen geeigneten Mittels.

10. Verfahren zur Bekämpfung von pflanzenpathogenen Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Ansprüchen 1 bis 6 behandelt.

## Revendications

1. Utilisation de phénéthylacrylamides de formule I 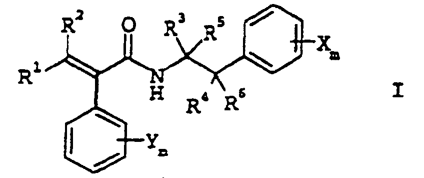 dans laquelle les substituants prennent la signification suivante :
X représente un atome d'halogène, un groupe alkyle en C₁ à C₄, halogénoalkyle en C₁ à C₄, alcoxy en C₁ à C₈, halogénoalcoxy en C₁ à C₄, et -O-C(R^{a}, R^{b})-C≡R^{c};
R^{a}, R^{b} représentent indépendamment l'un de l'autre, un atome d'hydrogène et un groupe alkyle en C₁ à C₆;
R^{c} représente un atome d'hydrogène, un groupe alkyle en C₁ à C₈, cycloalkyle en C₃ à C₈, et phényle, lequel peut être substitué par un atome d'halogène, un groupe cyano, nitro, CF₃, alkyle en C₁ à C₄, et/ou alcoxy en C₁ à C₄;
m vaut de 1 à 4, les résidus X pouvant être différents lorsque m est supérieur à 1;
n vaut 1 ou 2, les résidus Y pouvant être différents lorsque n vaut 2;
Y représente un atome d'halogène, un groupe nitro, cyano, alkyle en C₁ à C₄, CF₃, alcoxy en C₁ à C₄, et phényle, Y se trouvant dans la position 3, 4 ou 3,4;
R¹, R² représentent indépendamment l'un de l'autre, un atome d'hydrogène, un atome d'halogène, un groupe alkyle en C₁ à C₄, et CF₃;
R³, R⁴, R⁵, R⁶ représentent indépendamment l'un de l'autre, un atome d'hydrogène, un groupe alkyle en C₁ à C₄, et alcoxy en C₁ à C₄, ou bien
R³ et R⁴ forment ensemble un cycle cyclopropyle, les liaisons C-R⁵ et C-R⁶ pouvant être en configuration E ou Z, l'une par rapport à l'autre;
pour lutter contre les champignons phytopathogènes.

2. Phénéthylacrylamides de formule I selon la revendication 1, dans laquelle R¹ et R² sont identiques et représentent Cl, F et CH₃.

3. Phénéthylacrylamides de formule I selon les revendications 1 ou 2, dans laquelle m vaut 1 ou 2 et X se trouve dans la position 3, 4 ou 3,4.

4. Phénéthylacrylamides de formule I selon les revendications 1 à 3, dans laquelle X représente un groupe alcoxy en C₁ à C₈, et Y un atome d'halogène.

5. Phénéthylacrylamides de formule I selon les revendications 1 à 4, dans laquelle R³ et R⁴ représentent un atome d'hydrogène.

6. Phénéthylacrylamides de formules

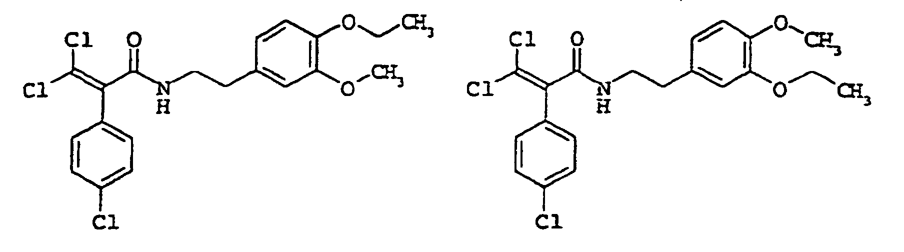

7. Procédé de préparation des composés selon les revendications 2 à 6, **caractérisé en ce que** l'on saponifie des α-cétoesters de formule II, 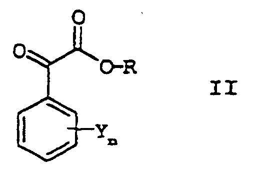 dans laquelle R représente un groupe alkyle en C₁ à C₄, avec la triphénylphosphine et, lorsque R¹ et R² représentent un atome de chlore, avec CCl₄, ou bien, lorsque R¹ et R² représentent un atome de fluor, avec le difluorochloracétate de sodium, ou bien, lorsque R¹ et R² représentent un groupe méthyle, dans des conditions basiques, avec un halogénure d'isopropyltriphénylphosphonium, en des esters acryliques de formule III 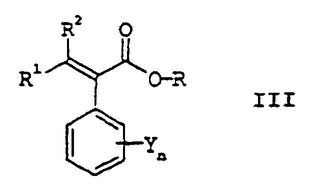 et III en des acides carboxyliques de formule IV 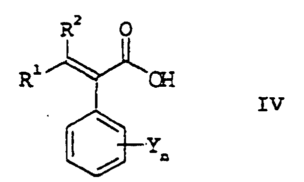 et on condense IV avec des phényléthylamines de formule V 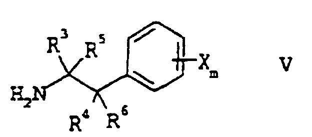 en composés de formule I.

8. Agent convenant à la lutte contre des champignons phytopathogènes, contenant une substance de support solide ou liquide, et un composé de formule I selon les revendications 2 à 6.

9. Utilisation des composés I selon les revendications 2 à 6, pour préparer un agent convenant à la lutte contre les champignons phytopathogènes.

10. Procédé pour lutter contre des champignons phytopathogènes, **caractérisé en ce que** l'on traite les champignons ou les matières, les plantes, le sol, ou les semences, à protéger contre une infection par les champignons, par une quantité efficace d'un composé de formule générale I selon les revendications 1 à 6.

## Claims

1. The use of phenethylacrylamides of the formula I 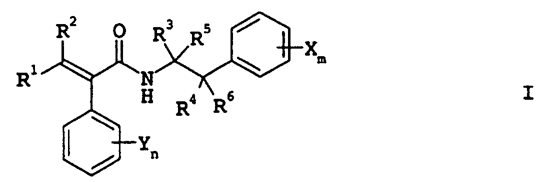 in which the substituents have the following meanings:
X is halogen, C₁-C₄-alkyl, C₁-C₄-haloalkyl, C₁-C₈-alkoxy, C₁-C₄-haloalkoxy and -O-C(R^{a},R^{b})-C≡C-R^{c};
R^{a},R^{b} independently of one another are hydrogen and C₁-C₆-alkyl;
R^{c} is hydrogen, C₁-C₈-alkyl, C₃-C₈-cycloalkyl and phenyl which can be substituted by halogen, cyano, nitro, CF₃, C₁-C₄-alkyl and/or C₁-C₄-alkoxy;
m is 1 to 4, it being possible for the radicals X to be different if m is greater than 1;
n is 1 or 2; it being possible for the radicals Y to be different if n is 2;
Y is halogen, nitro, cyano, C₁-C₄-alkyl, CF₃, C₁-C₄-alkoxy and phenyl, Y being in the 3-, 4- or 3,4-position;
R¹, R² independently of one another are hydrogen, halogen, C₁-C₄-alkyl and CF₃;
R³,R⁴,R⁵,R⁶ independently of one another are hydrogen, C₁-C₄-alkyl and C₁-C₄-alkoxy or
R³ and R⁴ together form a cyclopropyl ring, it being possible for the C-R⁵- and C-R⁶-bonds to be in the E- or Z-position relative to each other;
for controlling phytopathogenic fungal pests.

2. A phenethylacrylamide of the formula I as claimed in claim 1 wherein R¹ and R² are identical and are Cl, F and CH₃.

3. A phenethylacrylamide of the formula I as claimed in claim 1 or 2 where m is 1 or 2 and X is in the 3-, 4- or 3,4-position.

4. A phenethylacrylamide of the formula I as claimed in any of claims 1 to 3 where X is C₁-C₈-alkoxy and Y is halogen.

5. A phenethylacrylamide of the formula I as claimed in any of claims 1 to 4 where R³ and R⁴ are hydrogen.

6. A phenethylacrylamide of the formulae

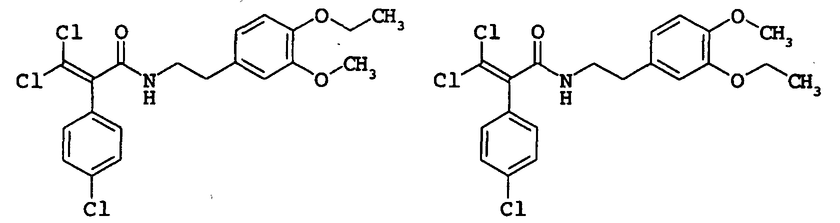

7. A process for the preparation of a compound of claims 2 to 6, which comprises [lacuna] α-keto esters of the formula II, 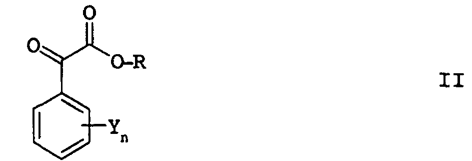 where R is C₁-C₄-alkyl, with triphenylphosphine and, if R¹ and R² are chlorine, with CCl₄, or, if R¹ and R² are fluorine, with sodium difluorochloroacetate, or, if R¹ and R² are methyl, with isopropyltriphenylphosphonium halide under basic conditions
to give acrylic esters of the formula III 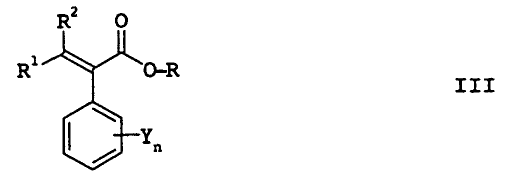 and hydrolyzing III to give carboxylic acids of the formula IV 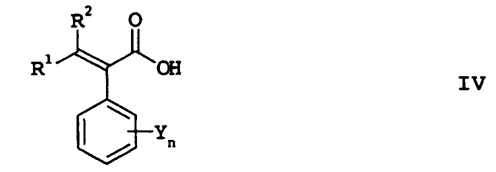 and subjecting IV to a condensation reaction with phenethylamines of the formula V 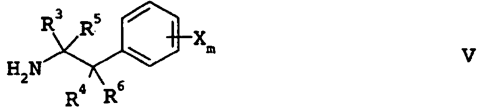 to give compounds of the formula I.

8. A composition which is suitable for controlling phytopathogenic harmful fungi, comprising a solid or liquid carrier and a compound of the formula I as claimed in any of claims 2 to 6.

9. The use of the compound I as claimed in any of claims 2 to 6 for preparing a composition which is suitable for controlling phytopathogenic harmful fungi.

10. A method of controlling phytopathogenic harmful fungi, which comprises treating the fungi or the materials, plants, the soil or seed to be protected from fungal infection with an effective amount of a compound of the formula I as claimed in any of claims 1 to 6.
